(19) Europäisches Patentamt

European Patent Office

Office européen des brevets

(11) **EP 1 625 841 A1**

(12) **EUROPEAN PATENT APPLICATION**
published in accordance with Art. 158(3) EPC

(43) Date of publication:
**15.02.2006 Bulletin 2006/07**

(51) Int Cl.:
*A61H 1/02* (1968.09)     *A61B 5/11* (1990.01)

(21) Application number: **04734349.6**

(22) Date of filing: **21.05.2004**

(86) International application number:
**PCT/JP2004/006940**

(87) International publication number:
**WO 2004/103244 (02.12.2004 Gazette 2004/49)**

(84) Designated Contracting States:
**DE**

(30) Priority: **22.05.2003 JP 2003145425**

(71) Applicants:
• **Hokkaido Technology Licensing Office Co., Ltd. Sapporo-Shi,
Hokkaido 060-0807 (JP)**
• **TOKAI UNIVERSITY EDUCATIONAL SYSTEM Shibuya-ku,
Tokyo 151-0063 (JP)**
• **Kyowa Electronic Instruments Co, Ltd Chofu-shi
Tokyo 1820021 (JP)**

(72) Inventors:
• **TANAKA, Toshiaki;
c/o Sapporo Medical
Sapporo-Shi, Chuo-Ku, Hokkaido;
0608556 (JP)**

• **IZUMI, Takashi;
c/o Hokkaido Tokai University,
Minami-Ku, Hokkaido;
0058601 (JP)**
• **TSURUGA, Takeshi;
c/o Hokkaido Instutute of
Sapporo-Shi, Teine-Ku, Hokkaido;
0068585 (JP)**
• **OMURA, Akinori;
c/o KYOWA ELECTRONIC INSTRUMENTS
Chofu-Shi, Tokyo 1820021 (JP)**

(74) Representative: **Wise, Stephen James et al
c/o RAWORTH, MOSS & COOK,
Raworth House,
36 Sydenham Road
Croydon,
Surrey CR0 2EF (GB)**

(54) **DEVICE AND METHOD OF APPLYING SKIN SENSORY STIMULATION**

(57)     An apparatus generates a cutaneous sense stimulus to call a subject attention to a change in posture based on posture change information (the amount of change in the barycenter, a changing direction of the same, and the like) from a device for detecting a change in posture of the subject. The apparatus has stimulus generating means (a plurality of vibrators) for generating a cutaneous sense stimulus, and stimulus control means for driving the stimulus generating means based on the posture change information. The control means comprises means for selecting a plurality of vibrators arranged substantially parallel with the changing direction based on the changing direction of the barycenter, and sequentially driving the selected vibrators, for example, in a direction substantially opposite to the direction in which the barycenter changes. The control means also drives the vibrators to generate the tactile sense stimulus of a magnitude corresponding to a changing amount of the barycenter. The vibrators are mounted on plantar soles, waist, chest, or the like. The apparatus can accurately notify the subject of a change in posture, and prompt the subject to take actions to maintain the posture.

FIG.3

Printed by Jouve, 75001 PARIS (FR)

**Description**

TECHNICAL FIELD

The present invention relates to an apparatus and method for applying a cutaneous sense stimulus, and more particularly, to technologies for allowing a human who has a handicap in balancing capabilities due to a disease or an injury to hold the position of the barycenter, and displacements/changing states of respective parts of the body in appropriate state when the human is trained to recover functions.

BACKGROUND ART

A human has balancing capabilities for holding a constant posture and a moving direction against the gravity, and moving associated parts of the body, when an inclination of the body has changed with respect to the direction of the gravity, to compensate for the change. However, such equilibrium/balancing capabilities can be degraded or lost due to an injury or some disease, for example, cerebrovascular accident, Parkinson's disease, labyrinth disorder or the like. In hospitals and a variety of medical related facilities , those humans who have such after-troubles are rehabilitated after a medical treatment stage has been over to recover the function of balancing capabilities.

As an apparatus for supporting such a function recovery training, there are, for example, those disclosed in JP-A-7-275307 and JP-A-9-120464. An apparatus described in JP-7-275307 detects the position of the barycenter of a subject in real time and visually displays the detected position, forcing the subject to move the barycenter such that his position of the barycenter overlaps a target image. An apparatus described in JP-A-9-120464 creates an image of a subject who is moving in a virtual space based on barycenter position data, and trains the subject to move the barycenter. Further, JP-A-2001-75705 describes an invention which utilizes a tactile sense to present a direction to a subject.

Disclosure of the Invention

By the way, when a human maintains a standing posture, a variety of factors affect the stability, where the width of the base of support is regarded as a main factor among them. The base of support is a concept which means the total area of both plantar soles and the ground between them when a human stands up by two legs, and a wider base of support results in a better stability (for example, in comparison of two legs in close contact with each other with two outstretched legs, the two outstretched legs increase the area between the two feet, thus resulting in a wider base of support and an increased stability).

On the other hand, when the balancing capabilities or their function recovery is taken into consideration, the relationship between the base of support and the barycentric line (position of the barycenter in a subject) plays an important role rather than the width of the base of support itself. This is because holding the equilibrium means maintaining the barycenter within the base of support with stability. Specifically, the stability is ameliorated as the position of the barycentric line is closer to the center of the base of support, and conversely, the stability is exacerbated as the position of the barycentric line is further away from the center of the base of support. If the barycenter fluctuates, causing its position to approach the edge of the base of support, the influence of even a slight external force causes the barycenter to deviate from the base of support, with the result that the subject turns over.

In comparison of an able-bodied person with a person who has any handicap in the equilibrium/balancing function, the former (able-bodied person) has the bary centric line maintained substantially at the center of the base of support. On the other hand, the latter (handicapped person) suffers from large fluctuations in the position of the barycentric line, which does not stay at the center of the base of support but grows to such an extent that the barycentric line may sometimes deviate from the base of support, causing the person to turn over.

Therefore, in the function recovery training, it is desirable to make the subject maintain the position of the barycentric line at the center of the base of support as stable as possible, and to promote the subject to spontaneously perform such actions.

However, the inventions described in the aforementioned JP-A-7-275307 and JP-A-9-120464 are intended to basically move the subject's barycenter to a predetermined target position, and are not provided for stabilizing the position of the barycenter or inducing such actions. Therefore, for example, for a person who unconsciously makes large changes in the posture, but is not aware by himself that he has lost the balance, the apparatuses described in these official gazettes experience difficulties in calling the person attention or avoiding the danger of a violent fall. Also, either of the apparatuses described in these official gazettes is such that a subject trains while viewing images, so that they need large-scaled devices such as a display, and do not directly act on the body of the subject to promote actions for maintaining the posture.

On the other hand, the invention described in the aforementioned JP-A-2001-75705 applies a direct tactile sense stimulus to the body surface to present a direction to a subject. However, this invention simply applies the stimulus to a certain point of the body, but does not detect a change in the subject's posture to let the subject know the change, or cannot prompt the subject to induce actions for maintaining the posture.

Further, not only in the scenario of the function recovery training as mentioned above, further improvements may be desired for able-bodied persons in the position of the barycenter, the posture, and the balance retention capabilities in a variety of situations. For example, in a variety of sports such as ski, skate, tennis, and golf, the position of the barycenter, and how to move the position can be main factors which govern the progress of skill. Also, for preventing a violent fall, for example, in walking on icy snow-covered roads, it is important to maintain the position of the barycenter in an appropriate state by shifting the weight in a proper manner.

It is therefore one of main objects of the present invention to maintain the "posture" in an appropriate state, in a broad meaning including the position of the barycenter, a displacement state of each part in the body, and the like, and particularly to prompt a subject himself to spontaneously perform actions and motions to maintain a proper posture or correct the posture.

To achieve the above object and solve the problem, a cutaneous sense stimulus applying apparatus according to the present invention is an apparatus for applying a cutaneous sense stimulus to a subject based on information on a change in posture of the subject outputted from posture change detecting means capable of detecting a change in posture of the subject, which comprises stimulus generating means for generating a cutaneous sense stimulus, and stimulus control means for driving the stimulus generating means based on the information on a change in posture.

The apparatus of the present invention generates a cutaneous sense stimulus which is applied to the subject, thereby calling the subject attention to a change in posture, and inducing (facilitating) or inhibiting desired motions or actions. Further, in addition to these actions, according to the apparatus of the present invention, muscles (or example, muscles weakened by a disease or a lesion) can be strengthened by repeating the operation based on the cutaneous sense stimulus, or a muscle hyper tonus can be loosened (mitigated or relaxed) by applying the cutaneous sense stimulus to the muscle, providing a massaging effect and a blood flow improving effect as well.

The cutaneous sense stimulus is generated by the stimulus generating means. The stimulus generating means is driven by the stimulus control means based on information on a change in posture outputted from posture change detecting means which is capable of detecting a change in posture of the subject.

In the present invention, the "posture" is a concept which widely includes the position of the barycenter, and the state of each part of the body (for example, arms, legs, trunk, waist, head and the like), while a "change in posture" refers to an action or a motion for changing the "posture" (for example, various motions of the body such as raising an arm or a leg, pulling the waist, bending, inclining and twisting the upper body, lowering the head, bending, stretching, turning (twisting) joints, and the like).

On the other hand, a "cutaneous sense" is a concept which includes not only a tactile sense, but also various senses associated with the human's skin such as a pressure sense, a thermal sense, a vibration sense, and a pain sense, and a "cutaneous sense stimulus" refers to a stimulus which can cause these senses. This stimulus is not necessarily limited to one which can be recognized by a subject himself, but also includes those which cannot be recognized by the subject himself (unconscious/reflex movement). This is because, for example, even when the cutaneous sense stimulus (for example, a vibratory stimulus), called in the present invention, is applied to a paralyzed body part of a subject caused, for example, by apoplectic stroke, but cannot be recognized by the subject himself, the direct action can be taken to muscles of the subject to prompt (facilitate) the subject to take actions for controlling the posture, or to inhibit undesirable body states, motions, and actions.

To accomplish such conscious or unconscious facilitating or inhibiting effects, the cutaneous sense stimulus preferably has a magnitude (intensity) with which the muscle itself can be stimulated, including a sensory receptor such as muscle spindle, a stimulus duration, a timing, a stimulated site, and the like.

Describing a specific example of the cutaneous sense stimulus, a vibratory stimulus (vibration) can be typically presented. However, the cutaneous sense stimulus, so called in the present invention, is not limited to the vibratory stimulus, but includes a variety of stimuli associated with the skin, for example, pressing the skin (applying a certain pressure for a certain period), pulling, giving a temperature difference between the skin, applying an electric stimulus (for example, attaching electrodes on the body), and the like.

The stimulus generating means preferably comprises a plurality of stimulus generator units such that the cutaneous sense stimulus can be applied to a plurality of points in part of the body of the subject. This is because, by sequentially driving a plurality of stimulus generator units to generate a continuous cutaneous sense stimulus (which appears to move or trace), facilitation (promoting actions or the like) and inhibition (loosening tense muscles or the like) can be more effectively carried out.

"Part of body (body part)" refers to part of the body, specifically, plantar sole, toe, leg (lower limb, femora), trunk, girth of abdomen (pelvis), hip (buttocks), arm (brachium, forearm), fingers, chest, neck, face, head, and the like, and the cutaneous sense stimulus is applied to a plurality of points of the body part (for example, a plurality of points on the plantar sole) by a plurality of stimulus generator units. Also, the body part called herein need not be necessarily one position, but a plurality of stimulus generator units may be provided for a plurality of parts of the body (for example, such as the plantar sole and trunk), respectively, to apply the cutaneous sense stimulus thereto.

The plurality of stimulus generator units are arranged, for example, in a substantially horizontal flat surface substantially perpendicular to the direction of the gravity. Also, the plurality of stimulus generator units may include stimulus generator units arranged at least in front of, at the back of, to the left of, and to the right of the position of the barycenter of the subject when the subject is stable in posture. Further, the stimulus control means may comprise selecting means for selecting a plurality of stimulus generator units arranged substantially parallel with a changing direction of the position of the barycenter based on information on a direction in which the position of the barycenter changes from the plurality of stimulus generator units to sequentially drive a plurality of selected stimulus generator units for generating a cutaneous sense stimulus. In this event, the driving order is set, for example, in substantially the opposite direction to the direction in which the position of the barycenter changes. This is because an action for pulling back the barycenter can be promoted by generating the cutaneous sense stimulus such that it moves in the direction opposite to the direction in which the barycenter changes.

The apparatus of the present invention may further comprise a base member for supporting the stimulus generating means and capable of directly or indirectly bringing the stimulus generating means into contact with the skin of the subject. Specifically, this base member is, for example, a shoe, an inner sole thereof, a strip-shaped member which can be wound around an arm, a leg, a trunk, and the like, an elastic member (fabric member, resin sheet, or the like) which can deform in conformity to the outer shape of a body part of the subject and come into close contact with the body surface, medical appliances, cushion, grip of stick, and the like. Also, the base member may comprise fixing means for fixing to the body surface of the subject. The fixing means can be implemented, for example, by a belt, a string, Velcro (laminating fastener, planar fastener), or an adhesive layer which can be adhered to the skin or clothing.

Also, a "direct or indirect contact" means not only a direct contact with the skin of the subject, but also an indirect contact with the skin of the subject, for example, through clothing, stocking, another member or material, or the like.

According to the apparatus which comprises the base member as described, the cutaneous sense stimulus, so called in the present invention, can be more readily applied to the subject, for example, by disposing the stimulus generating means in inner soles (base members) of shoes and placing the inner soles in existing shoes.

The information outputted from the posture change detecting means may include barycenter information on the position of the barycenter of the subject, and the stimulus control means may include changing amount calculating means for calculating the amount of a change in the position of the barycenter based on the barycenter information, and changing direction calculating means for calculating a changing direction of the position of the barycenter based on the barycenter information. This is intended to apply the subject with a cutaneous sense stimulus corresponding to the changing amount and changing direction, by detecting the changing amount and changing direction of the position of the barycenter.

The apparatus according to the present invention may further comprise comparing means for comparing the amount of change in the position of the barycenter with a previously set threshold, wherein the stimulus control means drives the stimulus generating means based on the result of the comparison by the comparing means. This is because, for example, when the position of the barycenter is monitored for movements to prevent turn-over, even a change in the position of the barycenter (even a change in posture) does not cause a need to drive the stimulus generating means for facilitation as is the case with the amount of change in the barycenter falling within an allowable range (a range without danger of fall).

Also, the stimulus control means may drive the stimulus generator units to generate the cutaneous sense stimulus of a magnitude corresponding to the changing amount based on the information on the amount of change in the position of the barycenter. According to such a configuration, the subject can be applied with the stimulus of a magnitude corresponding to the barycenter changing amount (i.e., the probability of turn-over), for example, when an attempt is made to prevent turn-over, to call the subject attention in accordance with the risk.

Further, the apparatus of the present invention may comprise drive display means which is capable of displaying a driving state of the stimulus generating means. According to such a configuration, an caregiver (helper), a medical staff (therapist), and a subject (patient or disabled person) can readily know a part which is applied with the stimulus, for example, when a patient or a handicapped person is rehabilitated, to more correctly keep track of a stimulus applying condition, and the physical condition of the subject.

Another cutaneous sense stimulus applying apparatus of the present invention comprises stimulus generating means for generating a cutaneous sense stimulus, a base member for supporting the stimulus generating means and capable of directly or indirectly bringing the stimulus generating means into contact with the skin of the subject, and stimulus control means for driving the stimulus generating means, wherein the stimulus generating means has a plurality of stimulus generator units such that the cutaneous sense stimulus can be applied to a plurality of points in part of the body of the subject, and the stimulus control means sequentially drives the plurality of stimulus generator units to generate the cutaneous sense stimulus.

According to the cutaneous sense stimulus applying apparatus as described above, by applying the subject with the cutaneous sense stimulus which moves on the body surface, in a manner similar to the aforementioned cutaneous sense stimulus applying apparatus, the subject can be prompted to take a desired motion/action or is provided with an inhibiting effect.

A cutaneous sense stimulus applying method according to the present invention includes a posture detecting step of detecting a change in posture of a subject, and outputting information on the change in posture, and a stimulus applying step for driving stimulus generating means capable of generating a cutaneous sense stimulus for application to the subject based on the outputted information on a change in posture to generate a cutaneous sense stimulus.

In the method as described, the stimulus generating means may comprise a plurality of stimulus generator units capable of generating the cutaneous sense stimulus, and the information on a change in posture may include information on the position of the barycenter of the subject. The method may further include a reference position measuring step of measuring the position of the barycenter of the subject when the subject is stable in posture, and a posture change calculating step of calculating a changing amount and a changing direction of the position of the barycenter based on the position of the barycenter when the subject is stable in posture, and the information on the position of the barycenter, and the stimulus applying step may include a comparing step of comparing the changing amount of the position of the barycenter with a previously set threshold, selecting a plurality of stimulus generator units for generating the cutaneous sense stimulus from the plurality of stimulus generator units based on the changing direction of the position of the barycenter , and a step of driving the plurality of stimulus generator units selected at the selecting step based on the result of the comparison at the comparing step.

According to the present invention as described above, a change in posture can be accurately informed, so that the subject can be prompted to spontaneously take actions for maintaining the posture. Other objects, features, and advantages of the present invention will become apparent from the following description of embodiments of the present invention.

BRIEF DESCRIPTION OF THE DRAWINGS

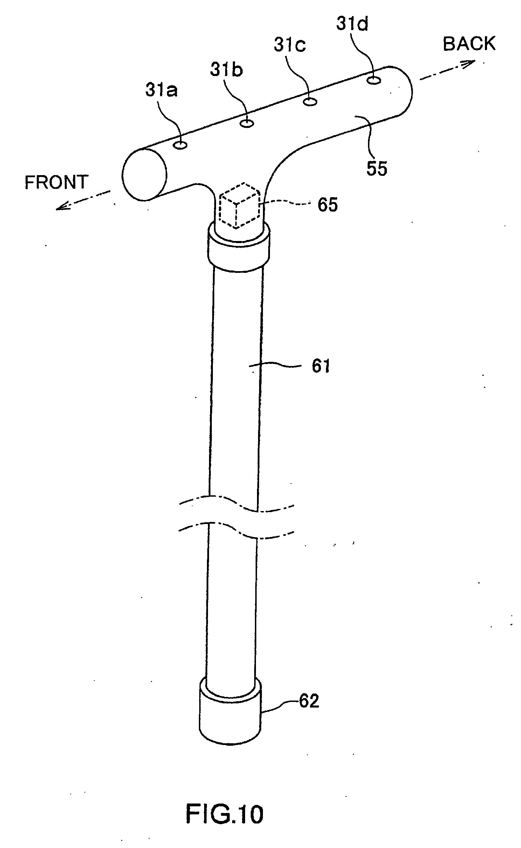

[Fig. 1]
A block diagram illustrating the configuration of a cutaneous sense stimulus applying apparatus according to one embodiment of the present invention.
[Fig. 2]
A conceptual diagram illustrating the configuration of the cutaneous sense stimulus applying apparatus according to the embodiment.
[Fig. 3]
Ablock diagram illustrating an exemplary system configuration of the cutaneous sense stimulus applying apparatus according to the embodiment.
[Fig. 4]
A plan view illustrating an exemplary layout of vibrators in the embodiment.
[Fig. 5]
A plan view illustrating another exemplary layout of vibrators in the embodiment.
[Fig. 6]
A diagram for describing a method of selecting and driving vibrators in the embodiment.
[Fig. 7]
A flow chart illustrating the operation of the cutaneous sense stimulus applying apparatus according to the embodiment.
[Fig. 8]
A diagram illustrating an exemplary arrangement of stimulus generating means (vibrators) in the present invention.
[Fig. 9]
Across-sectional view illustrating a cutaneous sense stimulus applying apparatus according to another embodiment of the present invention (an exemplary application to shoes).
[Fig. 10]
A perspective view illustrating a cutaneous sense stimulus applying apparatus according to another embodiment of the present invention, showing an exemplary arrangement of vibrators (stimulus generating means) in the apparatus (an example in which the vibrators are arranged in a walking stick).
[Fig. 11]
A diagram illustrating an exemplary modification to the cutaneous sense stimulus applying apparatus (walking stick) shown in Fig. 10, showing the bottom of its shoe.
[Fig. 12]
A diagram illustrating a cutaneous sense stimulus applying apparatus according to a further embodiment of the present invention, showing an exemplary arrangement of vibrators (stimulus generating means) in the apparatus (an example in which the vibrators are arranged on an arm).
[Fig. 13]
A diagram illustrating a cutaneous sense stimulus applying apparatus according to a further embodiment of the present invention, showing an exemplary arrangement of vibrators (stimulus generating means) in the apparatus (an example in which the vibrators are arranged on an arm in a similar manner).

BEST MODE FOR CARRYING OUT THE INVENTION

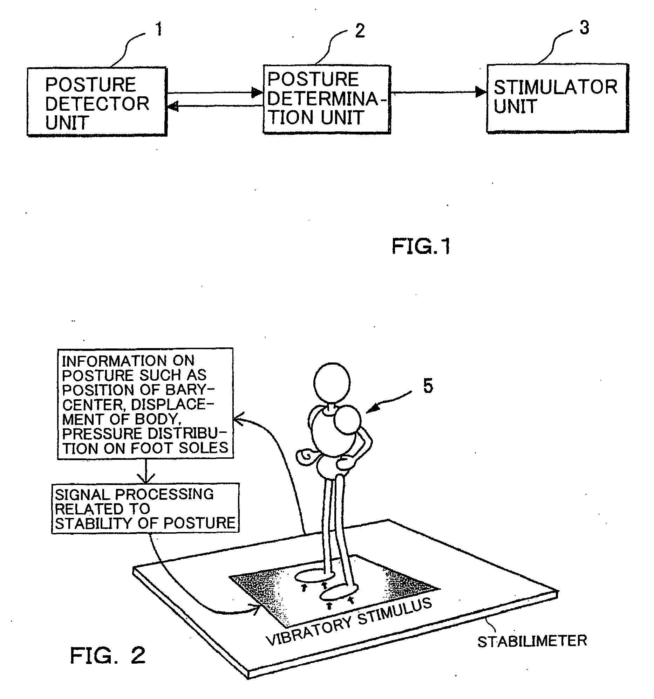

In the following, embodiments of the present invention will be described with reference to the accompanying drawings. As illustrated in Figs. 1 and 2, a cutaneous sense stimulus applying apparatus of this embodiment comprises a posture detector unit (posture change detecting means) 1 for collecting information on the posture of a subject 5; a stimulator unit (stimulus generating means) 3 for applying a cutaneous sense stimulus to the subject 5; and a posture determination unit (stimulus control means) 2 for determining a posture state of the subject 5 based on the information collected by the posture detector unit 1 to drive and control the stimulator unit 3.

Then, information on a change in posture (change in the barycenter) of the subject 5 is detected by the posture detector unit 1, and the posture determination unit 2 drives the stimulator unit 3 based on this information to apply a cutaneous sense stimulus to part of the body of the subject 5, thereby calling the subject attention to a change in posture and prompting the subject to return the posture to a stable state.

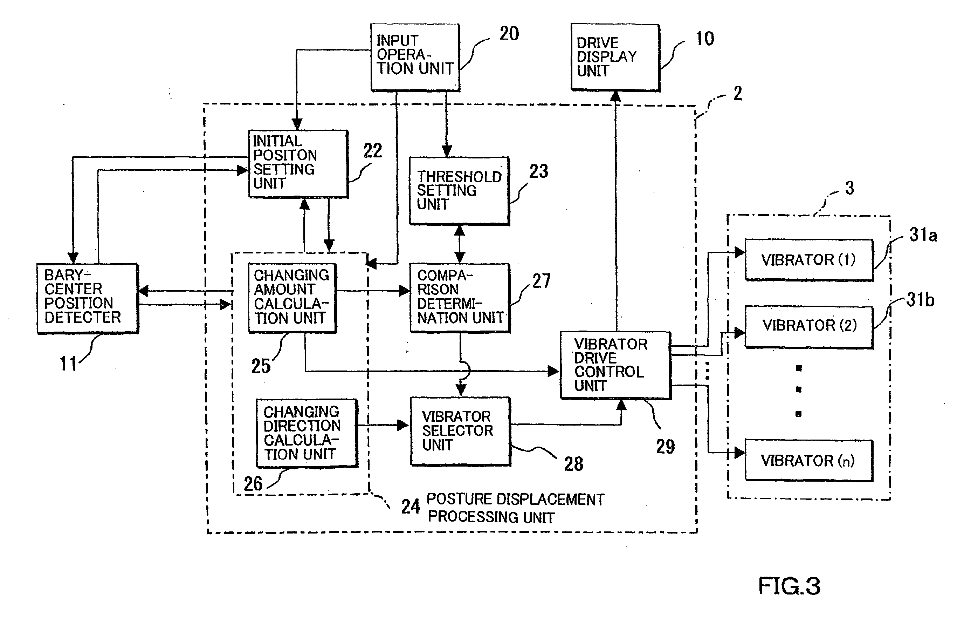

Specifically, the posture detector unit 1 can be implemented by a stabilimeter (barycenter oscillometer) for measuring the position of the barycenter of the subject 5 (for example, one which detects loads at three points by load cells to calculate the position of the barycenter, and the like), an inclination sensor capable of detecting an inclination, an acceleration sensor, a position sensor, a joint angle meter capable of measuring the angle of a joint in each site of the body, a motion analyzer, or the like, and can utilize these measuring devices which have been conventionally provided. Also, a plurality of these sensors may be used in combination. The posture determination unit 2 will be described later in greater detail with reference to Fig. 3 onward.

The stimulator unit 3 is preferably a member or an element which can apply a vibratory stimulus from a viewpoint of the ease of recognition by the subject and a more effective action to the body (muscle) of the subject. For example, the stimulator unit 3 can be implemented by a vibratory motor capable of applying a vibratory stimulus to the body surface of the subject (for example, a small vibratorymotor used inportable telephones canbe utilized) . However, the cutaneous sense stimulus generated in the present invention is not necessarily limited to the vibratory stimulus as mentioned above , but the "cutaneous sense stimulus" in the present invention includes various senses associated with the skin such as a pres sure sense caused, for example, by applying a constant pressure to the skin, and a thermal sense and a cold sense caused by a difference in temperature between the skin and surroundings, and further includes an electric stimulus which can be generated, for example, by mounting electrodes on the skin.

Referring to Fig. 3, the system configuration of this embodiment will be described in more specific manner. As illustrated in Fig. 3, this system comprises a barycenter position detector 11, a posture determination unit 2, and a stimulator unit 3, corresponding to the posture detector unit 1, posture determination unit 2, and stimulator unit 3 shown in the aforementioned Fig. 1, respectively, and further comprises an input operation unit 20 (for example, a keyboard, a numeral keyboard, a mouse, or the like) for performing input operations, and a drive display unit 10 for displaying a vibratory stimulus applying state by the stimulator unit 3 (driving state of the stimulator unit 3). Also, the transmission/reception of signals between respective components, for example, between the barycenter position detector 11 (posture detector unit 1) and the posture determination unit 2, or between the posture determination unit 2 and stimulator unit 3, and the like can be made in a wired manner through a transmission line, but may be made through radio communications as well. When the radio communication is used, signal transceivers should be provided as appropriate.

The barycenter position detector 11 can be implemented, for example, by a stabilimeter, as mentioned above, and the detector outputs barycenter position data of the subject as x-y coordinate values in real time.

On the other hand, the posture determination unit 2 comprises an initial position setting unit 22, a threshold setting unit 23, a posture displacement processing unit 24, a comparison determination unit 27, a vibrator selector unit 28, and a vibrator drive control unit 29. Among these components, the initial position setting unit 22 comprises a memory (not shown) for storing output data of the barycenter position detector 11, captures data from the barycenter position detector 11 through a predetermined input operation (for setting the position of the barycenter when the posture is stable) on the input operation unit 20, and stores this data in the memory as the position $(x0, y0)$ of the barycenter when the posture of the subject is stable.

The threshold setting unit 23 stores a threshold which serves as the basis for driving the stimulator unit 3 to apply a vibratory stimulus to the subject, and comprises a memory (not shown) for storing threshold data entered through the input operation unit 20. The threshold is set in this embodiment because the necessity arises to apply the vibratory stimulus to the subject to call his attention when there is a large change in posture (dangers such as a violent fall is predicted) . Therefore, the apparatus of this embodiment does not apply the vibratory stimulus when there is a few change in posture with a small amount of change in the position of the barycenter (when the amount of change is equal to or smaller than the threshold).

The posture displacement processing unit 24 comprises a changing amount calculation unit 25 and a changing direction calculating unit 26 for calculating a displacement of the position of the barycenter, and captures initial barycenter position data $(x_0, y_0)$ which is the position of the barycenter when the posture is stable, and current barycenter position data $(x, y)$ from the initial position setting unit 22 and barycenter position detector 11, respectively. Then, the changing amount calculation unit 25 calculates a changing amount $\underline{r}$ of the position of the barycenter based on the following equation (1) from these data, and outputs the data to the comparison determination unit 27 and vibrator drive control unit 29, respectively.

**[Equation 1]**

$$r = \sqrt{(x - x_0)^2 + (y - y_0)^2} \qquad \dots \quad (1)$$

On the other hand, the changing direction calculation unit 26 calculates a changing direction θ based on the following equation (2), and outputs the data to the vibrator selector unit 28.

**[Equation 2]**

$$\theta = \tan^{-1}\left(\frac{y - y_0}{x - x_0}\right) \qquad \dots \quad (2)$$

The comparison determination unit 27 captures data on the changing amount $\underline{r}$ of the position of the barycenter from the changing amount calculating unit 25, compares the data with a threshold $r_0$ inputted from the threshold setting unit 23, and outputs a selection command signal to the vibrator selector unit 28 when the changing amount $\underline{r}$ is larger than the threshold $r_0$.

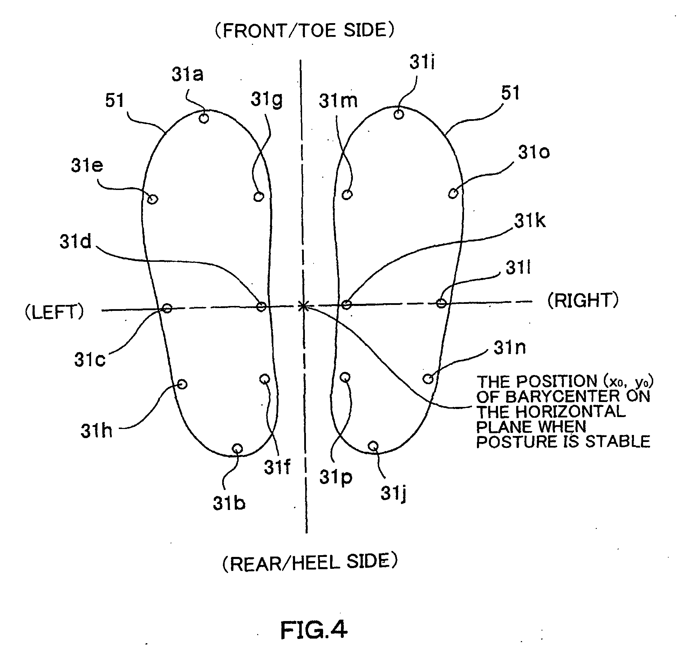

Based on the selection command signal, the vibrator selector unit 28 determines a vibrator 31a, 31b, ... within the stimulator unit 3 to be driven based on the output data θ from the changing direction calculation unit 26, and outputs a selection signal to the vibrator drive control unit 29. The vibrator drive control unit 29 drives a predetermined vibrator selected by the vibrator selector unit 28 with a magnitude corresponding to the changing amount $\underline{r}$ calculated by the changing amount calculating unit 25 (for example, with a magnitude proportional to the changing amount $\underline{r}$) based on the selection signal from the vibrator selector unit 28. Such a method of selecting and driving a vibrator will be described below in a specific manner with reference to Figs. 4 to 6.

As the posture detector unit 1, the barycenter position detector 11 can be replaced by an inclination sensor which is capable of detecting an inclination. The inclination sensor used herein should be capable of detecting the amount of inclination and an inclining direction, and the inclination sensor is mounted, for example, on the upper body (for example, on the back of the neck, back, chest, or the like) of the subject. An output signal from the inclination sensor includes data on the amount of inclination and inclining direction, where the inclination amount data is outputted to the comparison determination unit 27 through the posture displacement calculating unit 24, while the inclining direction data is outputted to the vibrator selector unit 28 through the posture displacement calculation unit 24. The threshold setting unit has previously set a certain amount of inclination (which may be determined as appropriate in accordance with the status of body, condition of handicap, and the like of the subject) as a threshold.

Subsequently, in a manner similar to the foregoing embodiment, the comparison determination unit 27 compares the amount of inclination inputted from the inclination sensor with the threshold, and outputs a selection command signal to the vibrator selector unit 28 when the amount of inclination is larger than the threshold. The vibrator selector unit 28 determines the vibrator 31a, 31b, ... within the stimulator unit 3 to be driven based on the inclination direction data, and outputs the selection signal to the vibrator drive control unit 29. The vibrator drive control unit 29 drives the selected predetermined vibrator with a magnitude corresponding to the amount of inclination based on the selection signal from the vibrator selector unit 28.

The inclination sensor maybe of a type which outputs a detection signal to the posture determination unit 2 only when the amount of inclination increases to a certain level or higher (when the application of vibratory stimulus is required). In this event, the detection signal is inputted to the vibration selector unit 28 (to the vibrator selector unit 28 and vibration drive control unit 29 when the detection signal includes information on the magnitude of the inclination), such that the vibrator selector unit 28 is configured to select a vibrator to be driven based on the detection signal. Therefore, according to this configuration, the comparison determination unit 27 and threshold setting unit 23 can be omitted.

Turning back to the embodiment of Fig. 3, the vibrator drive control unit 29 also outputs a driving signal to the vibrator 31a, 31b, ... selected by the vibrator selector unit 28, and also outputs a display signal indicative of the vibrator to be driven to the drive display unit 10. The drive display unit 10 comprises an image display means, for example, a liquid crystal display or the like, and displays a vibrator which is being driven in real time based on the display signal from the vibrator drive control unit 29.

A format for displaying on the display is not particularly specified. The vibrators may be displayed by a proper display method, for example, the state of arrayed vibrators may be visually displayed, while an actually (currently) driven vibrator may be blinked, filled with a color, displayed in a color different from vibrators which are not driven, and the like. While the drive display unit 10 is not an essential component in this embodiment and may be omitted, an apparatus comprising the drive display unit 10 advantageously allows a caregiver and a medical staff to immediately know how a vibration is being driven, i.e., how a cutaneous sense stimulus is being applied to the subject by viewing the display when the subject is rehabilitated.

The display signal from the vibrator drive control unit 29 maybe transmitted through a transmission line, i.e., in awiredfashion, but may be transmitted through a radio communication. In this event, a transmitter unit (not shown) is provided in the posture determination unit 2, while a signal receiver unit is provided in the drive display unit 10 for receiving signals transmitted from the transmitter unit. In this event, the drive display unit 10 may be configured as a dedicated device for directly receiving signals transmitted from the posture determination unit 2. Alternatively, a portable telephone or the like may be used to transmit the display signal through a public network or the like for display on a liquid crystal display.

The stimulator unit 3 comprises a plurality of vibrators (vibratory motors) 31a, 31b, .... These vibrators 31a, 31b, ... are mounted at predetermined sites on the body surface of the subject, and are driven by control signals from the vibrator drive control unit 29. The vibrators 31, 31b, ... can be arranged on a plantar sole, for example, by disposing them in an inner sole (base member) 51 of a shoe, as illustrated in Fig. 4. The vibrators are not particularly limited in number or mounted position. In this example, a total of 16 vibratory motors 31a - 31p are arranged in both the left and right shoes, i.e., a total of eight for each of the left and right shoes, four at a front, a rear, a left, and a right position, and another four off to the front left and right and off to the rear left and right of the shoe sole.

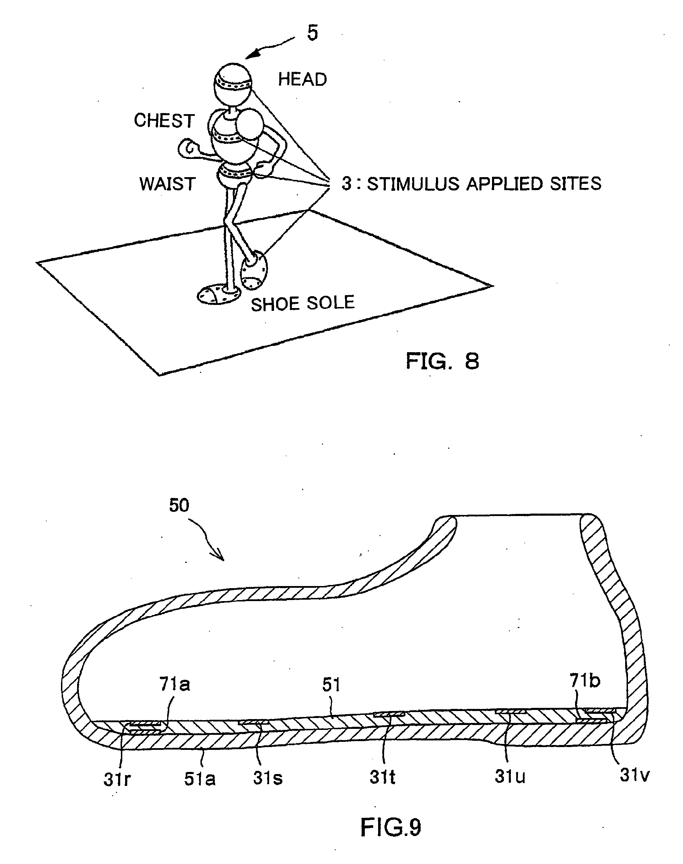

The sites of the body to which the vibrators are mounted can be selected as appropriate from the head, chest, waist, and other sites (for example, arms, femoral region, lower leg and the like), as illustrated in Fig. 8. For example, for a person who has lost a sense on the plantar sole, the cutaneous sense stimulus can be applied to the vibrators mounted on the chest, waist or the like, in which the cutaneous sense still remains, to call the person attention to a change in posture.

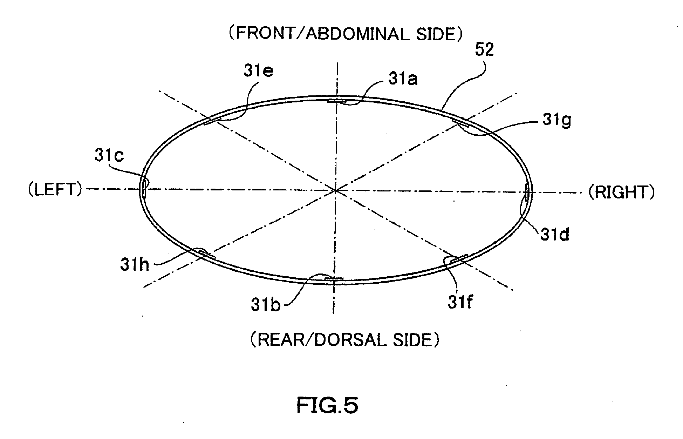

Fig. 5 illustrates an exemplary layout of vibrators mounted around the waist or chest. In this example, a total of eight vibratory motors 31a - 31h are disposed within a belt 52 which can be mounted around the waist or chest, and these motors are placed at a front, a rear, a left, and a right position, and off to the front left and right and off to the rear left and right of the belt.

While the vibrators can be mounted at a variety of sites of the body of the subject as described above, the vibrators are preferably placed at least at a front, a rear, a left, and a right position in a plane (horizontal plane) substantially perpendicular to the direction of the gravity (normal direction), as in the examples of Figs. 4 and 5 in order to call the person attention to a changing direction (to the front, rear, left, right and so on) of the barycenter.

A description will be given of a method of determining vibrators by the vibrator selector unit 28, and a method of driving the vibrators by the vibrator drive control unit 29.

The vibrator selector unit 28 in the apparatus of this embodiment sequentially selects vibrators arranged in a direction substantially parallel with the inclination of the posture of the subject (a change in the position of the barycenter). For example, when the position of the barycenter changes in front, vibrators arranged in a front-to-back direction parallel with this change are selected in order from the first one to the last one. On the other hand, when the barycenter changes in the left, vibrators arranged in the left-to-right direction in parallel with this change are selected in order from the one placed at the leftmost position to the one placed at the rightmost position.

Then, the vibrator drive control unit 29 sequentially drives the vibrators thus selected in the direction opposite to the barycenter changing direction to generate a cutaneous sense stimulus. For example, when the barycenter moves in front to result in the selection of the vibrators arranged in the front-to-back direction, a driving signal is sequentially output to each vibrator from the vibrator placed at the top position to the vibrator placed at the last position, to drive these vibrators one by one for a constant time from the front to the rear in order. In this way, the subject can be given a scene that the cutaneous sense stimulus moves continuously from the front to the rear.

While the number of vibrators to be driven can be one in the present invention, two or more vibrators arranged in a constant direction are preferably driven in sequence for generating such a continuous cutaneous sense stimulus.

Also, the magnitude of the vibrations to be generated is determined by the vibration drive control unit 29 based on the changing amount $\underline{r}$ calculated by the changing amount calculation unit 25. For example, when a voltage having a magnitude proportional to the changing amount $\underline{r}$ is applied to a vibratory motor to control the rotational speed thereof, the magnitude of resulting vibrations can be corresponded to the changing amount r. The relationship between the changing amount $\underline{r}$ and the magnitude of vibrations (a voltage supplied to the motor, or the like) is not necessarily limited to a proportional relationship. However, the magnitude of vibrations is preferably larger as the changing amount $\underline{r}$ is larger (positive correlation). This is because the risk of violent fall is higher as the changing amount $\underline{r}$ is larger, so that attention must be called more strongly.

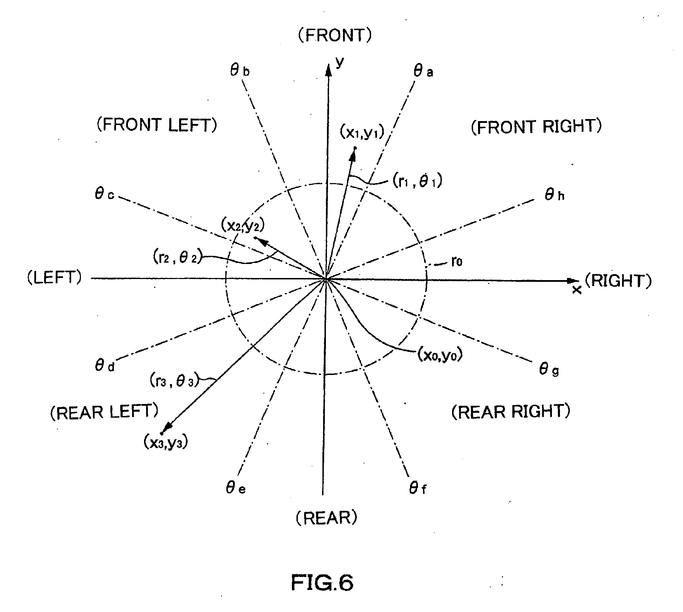

The method of selecting/driving vibrators will be described in a more specific manner. Fig. 6 illustrates x-y coordinates which represent the position of the barycenter (x, y) on the horizontal plane of a subject, where the origin of the coordinate system is made consistent with an initial position of the barycenter $(x_0, y_0)$. Also, for determining in which direction the posture of the subject is changing using a value θ indicative of a changing direction and for selecting a vibrator, the coordinate system is equally divided, for example, into eight directional zones, i.e., front ($\theta_a < \theta \leq \theta_b$), front left ($\theta_b < \theta \leq \theta_c$), left ($\theta_c < \theta \leq \theta_d$), rear left ($\theta_d < \theta \leq \theta_e$), rear ($\theta_e < \theta \leq \theta_f$), rear right ($\theta_f < \theta \leq \theta_g$), right ($\theta_g < \theta \leq \theta_h$), and front right ($\theta_h < \theta \leq \theta_a$).

The vibrator selector unit 28 compares changing direction data θ inputted from the changing direction calculating unit 26 with $\theta_a$ - $\theta_h$ to determine to which zone the changing direction θ of the barycenter belongs, thereby selecting a vibrator to be driven.

For example, assuming that the posture of the subject inclines in front to cause a displacement of the position of the barycenter to (x1, y1) (see Fig. 6), $\theta_a < \theta 1 \leq \theta_b$ is established in this case, so that the vibrator selector unit 28 selects the vibrators 31a, 31i which are arranged at the toe (front), and the vibrators 31b, 31j arranged at the heel (rear) (in the example of Fig. 5, the vibrator 31a on the abdominal side and the vibrator 31b on the dorsal side) in order, and outputs the selection signal to the vibrator drive control unit 29. The vibrator drive control unit 29 drives the vibrator 31a, 31i (vibrator 31a on the abdominal side in the example of Fig. 5) for a certain time, and then drives the vibrators 31b, 31j (vibrator 31b on the dorsal side in the example of Fig. 5) for a certain time based on the selection signal.

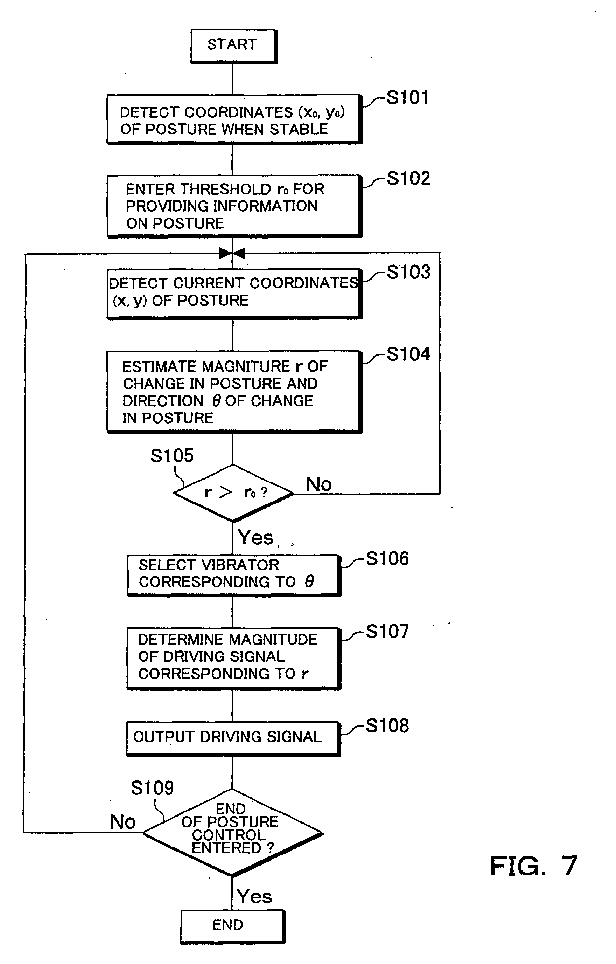

A driving time of each vibrator (how long it is driven), and a driving interval (a time interval from the time a certain vibrator is driven to the time the next vibrator is driven) can be set as appropriate in accordance with the age, the degree of handicap, the degree of remaining cutaneous sense, and the like of the subject, but these can be, for example, in a range of several tens of milliseconds to several hundred milliseconds. With this extent, a series of continuous vibratory stimuli can be applied once to several times for one to several seconds. As will be later described with reference to a flow chart of Fig. 7, the posture determination unit 2 captures barycenter position data of the subject from the barycenter position detector 11 at a predetermined sampling period (for example, at intervals of several milliseconds to several tens of milliseconds), and calculates the aforementioned changing amount $\underline{r}$ and changing direction θ to compare the changing amount $\underline{r}$ with the threshold $r_0$ and select vibrators, so that the continuous vibratory stimuli will be repeatedly applied to the subject unless the posture is returned (the changing amount $\underline{r}$ decreases to the threshold $r_0$ or less).

Likewise, for example, when the posture of the subject inclines diagonally to the rear left to change the position of the barycenter to (x3, y3) (see Fig. 6), $\theta_d < \theta 3 \leq \theta_e$ is established, so that the vibrator selector unit 28 orderly selects, in the example of Fig. 4, the vibrators 31h, 31p (vibrator 31h in the example of Fig. 5) in a diagonally rear left zone, and the vibrators 31g, 31o (vibrator 31g in the example of Fig. 5) in a diagonally front right zone. The vibrator drive control unit 29, based on the result of the selection, first drives the vibrators 31h, 31p (vibrator 31h) in the diagonally rear left zone, and then drives the vibrators 31g, 31o (vibrator 31g) in the diagonally front right zone. These vibrators are applied with a larger voltage, as compared with the position of the barycenter displaced to $(x_1, y_1)$, to generate stronger vibrations, because $r_3 > r_1$, as is apparent from the lengths of the vectors in Fig. 6.

Fig. 6 further illustrates a situation where the position of the barycenter of the subject is displaced to $(x_2, y_2)$. In this event, a changing amount $r_2$ is smaller than the threshold $r_0$, and therefore the comparison determination unit 27 will not output a selection command signal to the vibrator selector unit 28, so that the vibrator selector unit 28 will not output a selection signal to the vibrator drive control unit 29. Consequently, the vibrator drive control unit 29 does not output a driving signal to the stimulator unit 3 (vibrators 31a, 31b, ...), so that the subject is not applied with any vibratory stimulus.

In this embodiment, the method of driving (selecting) the vibrators 31 can be modified in various ways. For example, in an example in which the position of the barycenter is displaced to $(x_3, y_3)$ in the aforementioned Fig. 6, the vibrators 31h, 31p in the diagonally rear left zone are simultaneously driven for both the left and right feet, and then the vibrators 31g, 31o are driven for both the left and right feet in the same manner. In other words, the vibrators are driven (separately) for each of the left and right feet. Alternatively, the left and right feet may be collectively handled, and the vibrators may be driven such that the vibratory stimulus continuously flows from the left foot to the right foot, or from the right foot to the left foot.

For example, when the posture inclines to the left, the vibrators can be driven in the order of the vibrator 31c on the outer edge of the left foot - the vibrator 31d on the inner edge of the left foot - the vibrator 31k on the inner edge of the right foot → the vibrator 311 on the outer edge of the right foot, thus moving the vibratory stimulus gradually from the outside of the left foot to the outside of the right foot. Likewise, in the aforementioned example in which the position of the barycenter is displaced to $(x_3, y_3)$, the vibrators may be driven in the order of the left foot vibrator 31h - the left foot vibrator 31d → right foot vibrator 31k → right foot vibrator 31o instead of driving the vibrators separately for each of the left and right feet.

Further, in the foregoing example, the coordinate system is divided into eight angular zones (each zone extends over 45°) for determining the direction of a displacement of the barycenter based on the value of θ, but a smaller angular zones (for example, equally divided into ten, 16, or the like, or the coordinate system may not necessarily be divided into equal zones) may be set to correspond to vibrators which should be driven, or alternatively the coordinate system can be more roughly divided than the above (for example, the coordinate system may be equally divided into four to determine only the front, rear, left and right directions). However, for more accurately matching an actual direction of a displacement in posture with a direction in which a vibratory stimulus is presented, a larger number of vibrators are preferably provided to set smaller angular zones to which vibrators are corresponded.

The operation of the cutaneous sense stimulus applying apparatus according to this embodiment will be described with reference to Fig. 7. First, the position $(x_0, y_0)$ of the barycenter is detected when the subject is stable in posture (step S101), and a threshold $r_0$ is entered for a changing amount above which a vibratory stimulus should be applied (step S102). The threshold $r_0$ can be set as appropriate through the input operation unit 20, for example, in accordance with the degree of handicap, advancement of function recovery, and the like.

Next, a current position (x, y) of the barycenter is detected (step S103). The barycenter position detector 11 can detect the position (x, y) of the barycenter of the subject in real time, as previously mentioned. The posture determination unit 2 captures the barycenter position data (x, y) of the subject from the barycenter position detector 11 at a predetermined sampling period (for example, at intervals of several milliseconds to several tens of milliseconds), and the changing amount calculating unit 25 calculates a changing amount $\underline{r}$, while the changing direction calculating unit 26 calculates a changing direction θ, respectively (step S104).

Then, the comparison determination unit 27 compares the changing amount $\underline{r}$ with the threshold $r_0$ (step S105), and the vibrator selector unit 28 selects a vibrator 31 corresponding to θ if the changing amount $\underline{r}$ exceeds the threshold $r_0$ (step S106). Conversely, if the changing amount $\underline{r}$ is equal to or less than the threshold $r_0$, the flow returns to the aforementioned step S103 to detect the current coordinates of the posture. After the vibrator is selected (step S106), the vibrator drive control unit 29 determines the magnitude of a driving signal corresponding to the changing amount $\underline{r}$ (step S107), and outputs the signal to the selected vibrator (step S108). Then, the foregoing step S103 to step S108 are repeated until a signal for instructing the end of the posture control is entered from the input operation unit 20 (step S109).

According to the cutaneous sense stimulus applying apparatus of this embodiment, a cutaneous sense stimulus is applied to directly force the subject to control the posture, so that the subject can be more directly and effectively called attention than a method of forcing a subject to control the posture through the visual sense (for example, while viewing a screen). Also, the apparatus of the foregoing embodiment sequentially drives a series of a plurality of vibrators to apply a continuous cutaneous sense stimulus. Since this continuous stimulus flows in a direction opposite to a direction of a change in posture, the subject can be induced to imagine a scene of moving the barycenter along the flow of the continuous stimulus. It is therefore possible to guide the subject to spontaneously act to return to the original posture. By generating such a continuous cutaneous sense stimulus having the directivity, the apparatus of this embodiment can effectively force the subject to control the posture. Also, not only for handicapped persons and patients, but also for able-bodied persons, the posture, for example, can be correctly maintained by applying the cutaneous sense stimulus, thereby protecting/preventing lumbar pain, shoulder discomfort, and chronic lesions and diseases caused by an improper posture.

Further, the apparatus according to the present invention and this embodiment can be applied not only to the field of rehabilitation intended for the recovery of body functions, but also to exercises for moving the barycenter in a variety of sports, training and support for walking on snow-covered roads, training for improving body functions, intended for able-bodied persons, to enhance a balancing function, an equilibrium sense and the like, the presentation of a direction to the subject, and so forth. The apparatus can also provide muscle relaxation/massaging effect.

For example, when the inner sole illustrated in the aforementioned Fig. 4 can be arranged in shoes in a variety of sports such as ski, skate, golf, tennis and the like to apply a cutaneous sense stimulus, the subject can be informed and called attention when the position of the barycenter deviates from a desired range. Specifically, the threshold setting unit 23 in Fig. 3 may previously store data on a desired barycenter position range (of a skilled person, for example) to compare the stored data with the position of the barycenter of the subject detected by the barycenter position detector 11 in real time. Then, if the position of the barycenter of the subject deviates from the desired range for the barycenter, the vibrators may be driven to prompt the subject to stabilize the barycenter within the desired range.

In recent years, simulation apparatuses have been provided with regard to a variety of sports for projecting a play scene onto a screen to give a subject a feeling that he is actually playing. Thus, the aforementioned training (application of the vibratory stimulus) can be performed during actual plays, but may be used together with such a simulation apparatus. According to the simulation apparatus, since the subject himself does not move (does not actually coast down a slope or run about a tennis court), a stabilimeter installed on a floor can be used as the barycenter position detector 11.

Also, other than the sports, the apparatus according to the present invention and this embodiment can be utilized to train a person to walk or to support a person to walk on a snow-covered road and a frozen road.

Specifically, on a snow-covered road and an icy frozen road, when one rests his weight on part of the plantar sole (for example, on the heel), he is more susceptible to slip and turn-over. Therefore, in order to prevent this, when the position of the barycenter deviates from a certain range, a cutaneous sense stimulus can be applied to return it back, thereby training or supporting a person to walk.

In this event, pressure sensors are provided in addition to the vibrators in the base member (inner sole of a shoe) 51 of Fig. 4 to detect a loading condition, and the vibrators 31a - 31p are driven, when there is a certain deviation or more in the way of resting the weight (loading), to prompt the person to correct the deviation. The pressure sensors are disposed at least in a toe and a heel zone (additional pressure sensors may be provided on the left and right sides, for example, on the outside of the left foot and on the outside of the right foot) to detect a load at each point on the plantar sole. Then, the threshold setting unit 23 may previously store a predetermined load value determined based on the weight and the like of the subject as a threshold to determine an improper way of resting the weight when a pressure applied to the heel or toe reaches or exceeds the threshold, to drive the vibrators.

Further, Fig. 9 is a cross-sectional view illustrating an example of a shoe 50 for training or supporting a subject to walk, as mentioned above. This shoe 50 is provided with vibrators 31r - 31v on the surface of an inner sole 51 placed on a shoe sole 51a, and pressure sensors disposed on the lower surface of the inner sole 51. The pressure sensors include a sensor 71a disposed at a front position of the shoe, and a sensor 71b disposed at a rear position of the same. The positions at which the pressure sensors are disposed may not be on the lower surface of the inner sole, but may be on the upper surface or inside of the inner sole, on the shoe sole, or the like. Alternatively, instead of the pressure sensors, a switch (for example, a button switch which turns on when it is applied with a certain load or more) may be provided to detect a load through on/off of the switch, to drive the vibrators based on the detection.

A specific method of driving the vibrators, for example, when a load equal to or larger than the threshold is detected in a heel zone, drives the vibrator 31a or 31i at the toe in the example of Fig. 4 (for example, 31r in the example of Fig. 9) to prompt the subject to rest the weight on the toe as well. Conversely, when a load equal to or larger than the threshold is detected in a toe zone, the vibrator 31b or 31j at the heel (for example, 31v in the example of Fig. 9) is driven to prompt the subject to rest the weight on the heel as well. Further, in this event, the vibrators are preferably driven in sequence from the toe to the heel, like the aforementioned embodiment , or from the heel to the toe to apply a continuous stimulus which appears to flow for prompting the subject to take actions to move the barycenter.

When no load is detected either on the toe or on the heel, the vibrators may not be driven on the assumption that the foot is raised. This is because the foot on the ground can arise a problem particularly for preventing slip and turn-over during walking. Also, for detecting a raised foot during walking to avoid the application of vibrations to the raised foot, a sensor for detecting the angle of a joint may be relied on, instead of the pressure sensor and the like. For example, a sensor may be provided for sensing the angle of a knee, such that when the knee is bent at a certain angle or more, it is determined that this leg is not on the ground but is raised from the ground for stepping, in which case the vibrators are not driven.

Further, not only the legs, but a walking stick, when used, can cause a danger if it is pushed obliquely against the ground, making the subject more prone to slip.

Therefore, an apparatus illustrated in Fig. 10 comprises vibrators (stimulus generating means) in a grip 55 of a walking stick to apply a cutaneous sense stimulus to a palm. In the illustrated example, four vibrators 31a - 31d are arranged along the longitudinal direction of the grip 55 which extends in the front-to-back direction, and an inclination sensor 65 is additionally provided within the grip 55 or within a stick body 61 as means for detecting that the stick inclines obliquely from a perpendicular state.

In this apparatus, for example, when a shoe 62 at the leading end of the walking stick goes excessively in front so that the walking stick inclines too much in the rear, the inclination sensor 65 detects this inclination, and the vibrator 31d, vibrator 31c, vibrator 31b, and vibrator 31a are driven in order to prompt the subject to move the grip 55 in front or to again push the walking stick against the ground so that the walking stick is returned to be closer to the state perpendicular to the ground. Conversely, when the grip 55 is positioned excessively in front so that the walking stick inclines too much in front, the vibrator 31a, vibrator 31b, vibrator 31c, and vibrator 31d are driven in order to prompt the subject to pull back the grip 55. The number of vibrators and driving method may be a different number and a different driving order.

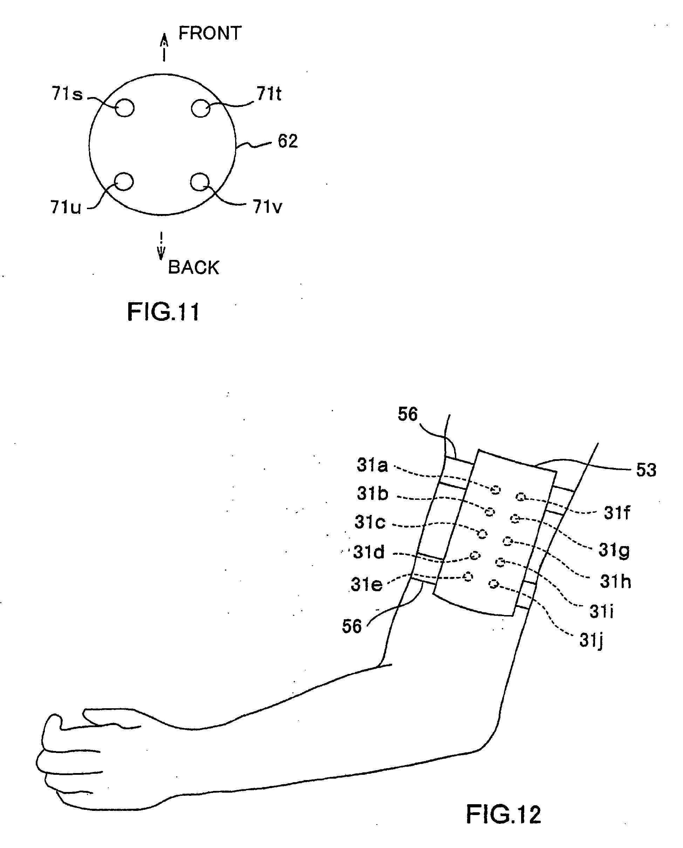

Further, this walking stick may not rely on the inclination sensor 65, but may comprise pressure sensors 71s - 71v (or switches) on the bottom of the shoe 62 (or between the stick body 61 and shoe 62) as illustrated in Fig. 11 to detect a load (or utilizing on/off of the switches), in a manner similar to the aforementioned shoe 50, to drive the vibrators 31a - 31d. Alternatively, a variety of sensors , for example, an acceleration sensor, a position sensor and the like may be used instead of the inclination sensor and pressure sensors, or in addition to the inclination sensor and pressure sensors, and a plurality of types of these sensors may be used in combination as well.

Also, a guiding apparatus can also be implemented for guiding a walking course, making use of the grip 55 of the foregoing walking stick, or vibrators disposed in the inner sole 51 of the shoe 50 as described above or a base member mountable to the body. Specifically, self position detecting means such as GPS may be provided as the posture detector unit 1 (Fig. 1) such that positional information is acquired in real time by the self position detecting means when surroundings are not clearly visible on a road at night, and the vibrators can be driven to apply a cutaneous sense stimulus to the trunk or legs and arms, thereby leading the subject in a desired direction to a destination.

Further, by applying the cutaneous sense stimulus as referred to in the present invention, it is possible to produce such effects as recovery of functions of a paralyzed body part, relaxation of muscles from unnecessary strain, massaging, and the like.

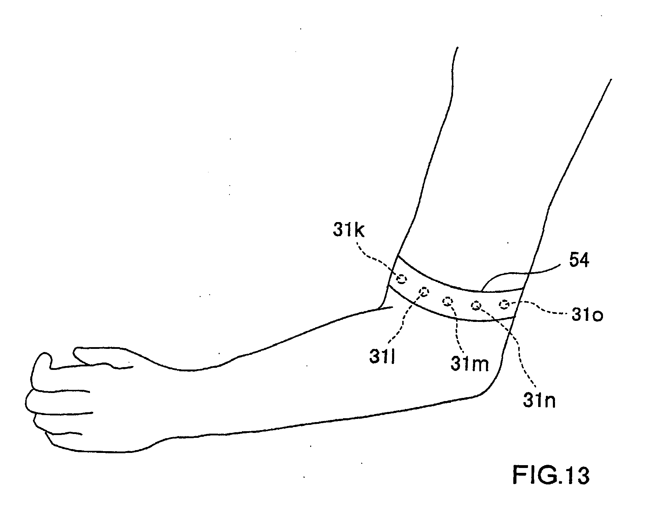

Figs. 12 and 13 illustrate examples of disposing vibrators for applying the cutaneous sense stimulus (vibratory stimulus) according to the present invention on a brachium, respectively. The example of Fig. 12 disposes a plurality (ten in this example) of vibrators 31a - 31j in a sheet-shaped base member 53 as the stimulator unit 3 (Fig. 1) for applying the vibratory stimulus to muscles of the brachium. The base member 53 which supports the vibrators is formed of an elastic material (for example, fabric, resin sheet or the like) such that the respective vibrators 31a - 31j can be brought into close contact with the body surface of the subject. Also, the base member 53 comprises a belt 56 which can be wound around an arm as fixing means for fixing the base member 53 on the arm of the subject. The fixing means may be, for example, a string, an adhesive layer formed on the back surface of the base member, or the like, other than the belt.

A method of driving the vibrators 31a - 31j (driving pattern) can take a variety of driving pattern such as (1) a pattern for sequentially driving them such that they flow in one direction; (2) a pattern for sequentially driving them such that they extend from a central region to the left and right; (3) a pattern for driving them such that they flow in one direction and then return in the opposite direction (reciprocal driving); (4) a pattern for sequentially driving them such that they swirl; (5) a pattern for driving at an arbitrary fixed point.

Describing more specifically, the driving pattern (1) drives , for example, in the order of vibrators 31a, 31b, 31c, 31d, 31e. Also, in this event, the vibrators 31f, 31g, 31h, 31i, 31j may be simultaneously driven (31a and 31f, 31b and 31g, 31c and 31h, 31d and 31i, 31e and 31j are simultaneously driven, respectively), and they may be sequentially driven in the opposite direction.

The driving pattern (2) firstdrives, for example, the vibrator 31c, and then 31b and 31d, and next 31a and 31e. In this event, the vibrators 31f - 31j may be simultaneously driven in a similar manner. The driving pattern (3) drives, for example, in the order of vibrators 31a, 31b, 31c, 31d, 31e, and then drives in the order of 31e, 31d, 31c, 31b, 31a such that the flow goes backward. This may be repeated. The vibrators 31f - 31j may also be simultaneously driven in a similar manner.

The driving pattern (4) drives, for example, in the order of the vibrators 31a, 31b, 31c, 31d, 31e, 31j, 31i, 31h, 31g, 31f, 31a, .... The rotating direction may be opposite. Thedrivingpattern (5) drives any of the vibrators (for example, only 31c, 31h).

On the other hand, the example of Fig. 13 comprises a plurality of vibrators in a strip-shaped base member 54 as the stimulator unit 3 (Fig. 1), such that the vibrators can be arranged around an arm (the figure shows only vibrators 31k - 31o on the outer side of the arm). A method of driving the vibrators may drive them, for example, in the clockwise direction or counter-clockwise direction around the arm.

When a moving stimulus is applied by a plurality of vibrators in the foregoing manner, for example, in rehabilitation of a patient who has contracted a disease such as apoplectic stroke which is associated with paralysis as a later effect, a paralyzed muscle can be recovered. Also, the moving stimulus can mitigate a muscle hyper tonus, relax muscles, and provide a massaging effect. The application of such a vibratory stimulus can be applied not only to arms but also to each part of the body such as legs, trunk and the like as well. Also, the cutaneous sense stimulus applying apparatus as illustrated in Figs. 12 and 13 does not necessarily require the posture detector unit 1 which acquires information on the posture of a subject, as shown in Fig. 1.

It is apparent to those skilled in the art that the present invention is not limited to the embodiments described with reference to the drawings, but can be modified in a variety of ways within the scope described in the claims.

For example, the number and layout pattern of the stimulus generatingmeans (vibrators), a site of the body for mounting, a specific driving pattern by stimulus control means, and the like can be modified in a variety of manners. For example, the apparatus for calling a subject attention to a change in posture can: (1) sequentially drive vibrators in a direction substantially matching a direction in which the position of the barycenter changes (in the forward direction) to apply a vibratory stimulus; (2) simultaneously drive a plurality of vibrators (for example, in the embodiment of Fig. 4, only the vibrators 31a, 31i, 31g, 31m, 31e, 31o in a front foot zone are simultaneously driven, or only the vibrators 31b, 31j, 31f, 31p, 31h, 31n in the rear foot zone are simultaneously driven, or the like) ; (3) collect a plurality of vibrators into groups, and sequentially drive the vibrators in units of groups (for example, in Fig. 4, the vibrators 31a, 31i, 31g, 31m, 31e, 31o in the front foot zone are first driven, followed by driving of the vibrators 31b, 31j, 31f, 31p, 31h, 31n in the rear foot zone); and the like.

Also, the respective components of the embodiments described as functional blocks in the description of the embodiments can be implemented in software or firmware by a computer program.

Further, the present invention can be applied to a subject under dynamic conditions (the barycenter moves) such as during walking, as well as to a static standing posture. Also, the present invention can be applied to shoes, walking sticks (cane), caster walkers, wheel chairs, artificial limbs, and assistive device for locomotion /supporting welfare equipment such as a variety of appliances, and according to the present invention, the cutaneous sense stimulus is utilized to provide information on the posture in a standing/walking training and the like, thereby facilitating a posture control.

Description of Reference Numerals

    1 Posture Detector Unit
    2 Posture Determination Unit
    3 Stimulator Unit
    5 Subject
    11 Barycenter Position Detector
    20 Input Operation Unit
    22 Initial Position Setting Unit
    23 Threshold Setting Unit
    24 Posture Displacement Calculating Unit
    25 Changing Amount Calculating Unit
    26 Changing Direction Calculating Unit
    27 Comparison Determination Unit
    28 Vibrator Selector Unit
    29 Vibrator Drive Control Unit
    31a - 31p, 31r - 31v Vibrators
    50 Shoe
    51 Inner Sole (Base Member)
    53 Sheet-Shaped Base Member
    54 Strip-Shaped Base Member
    55 Grip
    56 Belt
    61 Stick Body
    62 Shoe
    65 Inclination Sensor
    71a, 71b, 71s - 71v Pressure Sensors

**Claims**

1. An apparatus for applying a cutaneous sense stimulus to a subject based on information on a change in posture of the subject outputted from posture change detecting means capable of detecting a change in posture of the subject, said apparatus comprising:

    stimulus generating means for generating a cutaneous sense stimulus; and
    stimulus control means for driving said stimulus generating means based on the information on a change in posture.

2. A cutaneous sense stimulus applying apparatus according to claim 1, wherein:

    said stimulus generating means comprises a plurality of stimulus generator units such that the cutaneous sense stimulus can be applied to a plurality of points in part of the body of the subject.

**3.** A cutaneous sense stimulus applying apparatus according to claim 1 or 2, further comprising:

a base member for supporting said stimulus generating means and capable of directly or indirectly bringing said stimulus generating means into contact with the skin of the subject.

**4.** A cutaneous sense stimulus applying apparatus according to any of claims 1 to 3, wherein:

the information outputted from said posture change detecting means includes barycenter information on the position of the barycenter of the subject, and
said stimulus control means includes changing amount calculating means for calculating the amount of a change in the position of the barycenter based on the barycenter information, and changing direction calculating means for calculating a changing direction of the position of the barycenter based on the barycenter information.

**5.** A cutaneous sense stimulus applying apparatus according to claim 4, further comprising comparing means for comparing the amount of change in the position of the barycenter with a previously set threshold,
wherein said stimulus control means drives said stimulus generatingmeans based on the result of the comparison by said comparing means.

**6.** A cutaneous sense stimulus applying apparatus according to claim 4 or 5, wherein:

said stimulus control means drives said stimulus generator units to generate the cutaneous sense stimulus of a magnitude corresponding to the changing amount based on the information on the amount of change in the position of the barycenter.

**7.** A cutaneous sense stimulus applying apparatus according to any of claims 1 to 6, wherein:

said stimulus generating means comprises a plurality of stimulus generator units such that the cutaneous sense stimulus can be simultaneously applied to a plurality of points in part of the body of the subject, and
said plurality of stimulus generator units are arranged in a substantially flat surface substantially perpendicular to the direction of gravity.

**8.** A cutaneous sense stimulus applying apparatus according to any of claims 1 to 7, wherein:

said stimulus generating means comprises a plurality of stimulus generator units such that the cutaneous sense stimulus can be applied to a plurality of points in part of the body of the subject, and includes said stimulus generator units arranged at least in front of, at the back of, to the left of, and to the right of the position of the barycenter of the subject when the subject is stable in posture.

**9.** A cutaneous sense stimulus applying apparatus according to any of claims 1 to 8, wherein:

said stimulus generating means comprises a plurality of stimulus generator units such that the cutaneous sense stimulus can be applied to a plurality of points in part of the body of the subject, and
said stimulus control means comprises selecting means for selecting a plurality of stimulus generator units arranged substantially parallel with a changing direction of the position of the barycenter based on information on a direction in which the position of the barycenter changes from said plurality of stimulus generator units, and sequentially drives a plurality of selected stimulus generator units to generate a cutaneous sense stimulus.

**10.** A cutaneous sense stimulus applying apparatus according to claim 9, wherein:

said stimulus control means sequentially drives said plurality of stimulus generator units selected by said selecting means in a direction substantially opposite to the direction in which the position of the barycenter changes.

**11.** A cutaneous sense stimulus applying apparatus according to any of claims 1 to 10, wherein said stimulus generating means applies a cutaneous sense stimulus to the plantar soles of the subject.

**12.** A cutaneous sense stimulus applying apparatus according to claim 11, wherein:

said stimulus generating means is arranged in a shoe.

13. A cutaneous sense stimulus applying apparatus according to any of claims 1 to 10, wherein:

said stimulus generating means is arranged in a grip of a walking stick.

14. A cutaneous sense stimulus applying apparatus according to any of claims 1 to 13, further comprising:

drive display means for displaying a driving state of said stimulus generating means.

15. A cutaneous sense stimulus applying apparatus comprising:

stimulus generating means for generating a cutaneous sense stimulus;
a base member for supporting said stimulus generating means and capable of directly or indirectly bringing said stimulus generating means into contact with the skin of the subject; and
stimulus control means for driving said stimulus generating means,
wherein said stimulus generating means has a plurality of stimulus generator units such that the cutaneous sense stimulus can be applied to a plurality of points in part of the body of the subject, and
said stimulus control means sequentially drives said plurality of stimulus generator units to generate the cutaneous sense stimulus.

16. A cutaneous sense stimulus applying apparatus according to claim 3 or 15, wherein:

said base member has elasticity so as to be deformable in conformity to the outer shape of a bodypart of the subject, and comprises a fixing means capable of fixing said base member to the body part.

17. A cutaneous sense stimulus applying method comprising:

a posture detecting step of detecting a change in posture of a subject, and outputting information on the change in posture; and
a stimulus applying step for driving stimulus generatingmeans capable of generating a cutaneous sense stimulus for application to the subject based on the outputted information on a change in posture to generate a cutaneous sense stimulus.

18. A cutaneous sense stimulus applying method according to claim 17, wherein:

said stimulus generating means comprises a plurality of stimulus generator units capable of generating the cutaneous sense stimulus,
said information on a change in posture includes information on the position of the barycenter of the subject,
said method further includes:

a reference position measuring step of measuring the position of the barycenter of the subject when the subject is stable in posture ; and
a posture change calculating step of calculating a changing amount and a changing direction of the position of the barycenter based on the position of the barycenter when the subject is stable in posture, and the information on the position of the barycenter, and
said stimulus applying step includes:

a comparing step of comparing the changing amount of the position of the barycenter with a previously set threshold;
selecting a plurality of stimulus generator units for generating the cutaneous sense stimulus from said plurality of stimulus generator units based on the changing direction of the position of the barycenter; and
a step of driving the plurality of stimulus generator units selected at said selecting step based on the result of the comparison at said comparing step.

POSTURE
DETECTOR
UNIT
1

POSTURE
DETERMINA-
TION UNIT
2

STIMULATOR
UNIT
3

FIG.1

INFORMATION ON
POSTURE SUCH AS
POSITION OF BARY-
CENTER, DISPLACE-
MENT OF BODY,
PRESSURE DISTRIBU-
TION ON FOOT SOLES

SIGNAL PROCESSING
RELATED TO
STABILITY OF POSTURE

5

VIBRATORY STIMULUS

STABILIMETER

FIG. 2

FIG.3

(FRONT/TOE SIDE)

31a  31i
51  31g  31m  51
31e  31o
31d  31k
31l
(LEFT) ——————————— ✳ ——————————— (RIGHT)
31c  31n
31h  THE POSITION $(x_0, y_0)$
OF BARYCENTER ON
THE HORIZONTAL
PLANE WHEN
POSTURE IS STABLE
31f  31p  31j
31b

(REAR/HEEL SIDE)

FIG.4

(FRONT/ABDOMINAL SIDE)

(LEFT) — (RIGHT)

(REAR/DORSAL SIDE)

FIG.5

FIG.6

```
                    ┌─────────────┐
                    │    START    │
                    └─────────────┘
                           │
          ┌────────────────────────────────┐
          │ DETECT COORDINATES (x₀, y₀)     │──S101
          │ OF POSTURE WHEN STABLE          │
          └────────────────────────────────┘
                           │
          ┌────────────────────────────────┐
          │ ENTER THRESHOLD r₀ FOR          │──S102
          │ PROVIDING INFORMATION           │
          │ ON POSTURE                      │
          └────────────────────────────────┘
                           │
          ┌────────────────────────────────┐
          │ DETECT CURRENT COORDINATES      │──S103
          │ (x, y) OF POSTURE               │
          └────────────────────────────────┘
                           │
          ┌────────────────────────────────┐
          │ ESTIMATE MAGNITURE r OF         │──S104
          │ CHANGE IN POSTURE AND           │
          │ DIRECTION θ OF CHANGE           │
          │ IN POSTURE                      │
          └────────────────────────────────┘
                           │
    S105              ╱─────────╲       No
                     ⟨  r > r₀ ? ⟩────────
                      ╲─────────╱
                           │ Yes
          ┌────────────────────────────────┐
          │ SELECT VIBRATOR                 │──S106
          │ CORRESPONDING TO θ              │
          └────────────────────────────────┘
                           │
          ┌────────────────────────────────┐
          │ DETERMINE MAGNITUDE             │──S107
          │ OF DRIVING SIGNAL               │
          │ CORRESPONDING TO r              │
          └────────────────────────────────┘
                           │
          ┌────────────────────────────────┐
          │ OUTPUT DRIVING SIGNAL           │──S108
          └────────────────────────────────┘
                           │
    S109              ╱─────────╲
          No         ⟨  END      ⟩
         ────────────⟨ OF POSTURE ⟩
                     ⟨  CONTROL   ⟩
                     ⟨ ENTERED ?  ⟩
                      ╲─────────╱
                           │ Yes
                    ┌─────────────┐
                    │     END     │
                    └─────────────┘
```

**FIG. 7**

21

5

CHEST

HEAD

WAIST

3 : STIMULUS APPLIED SITES

SHOE SOLE

FIG. 8

50

71a          51          71b

31r    51a    31s        31t        31u        31v

FIG.9

31a  31b  31c  31d

BACK

FRONT

55

65

61

62

**FIG.10**

FRONT

71s — 71t

62

71u — 71v

BACK

FIG.11

56

31a — 53
31b
31c
31d — 31f
31e — 31g

56 — 31h

31i

31j

FIG.12

FIG.13

<table>
<tr><td colspan="2">**INTERNATIONAL SEARCH REPORT**</td><td>International application No.<br>PCT/JP2004/006940</td></tr>
</table>

| A. CLASSIFICATION OF SUBJECT MATTER |
|---|
| Int.Cl$^7$  A61H1/02, A61B5/11 |

According to International Patent Classification (IPC) or to both national classification and IPC

| B. FIELDS SEARCHED |
|---|

Minimum documentation searched (classification system followed by classification symbols)
Int.Cl$^7$  A61H1/02, A61H3/00, A61B5/11, A63B23/04, A43B3/00, A43B13/00,
A61H23/02, A61H39/04, G06F3/00

Documentation searched other than minimum documentation to the extent that such documents are included in the fields searched
| Jitsuyo Shinan Koho | 1926–1996 | Toroku Jitsuyo Shinan Koho | 1994–2004 |
| Kokai Jitsuyo Shinan Koho | 1971–2004 | Jitsuyo Shinan Toroku Koho | 1996–2004 |

Electronic data base consulted during the international search (name of data base and, where practicable, search terms used)

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | JP 8-141025 A  (Hitachi, Ltd.),<br>04 June, 1996 (04.06.96),<br>Full text; all drawings<br>(Family: none) | 1-3<br>4-16 |
| Y | JP 7-275307 A  (Anima Kabushiki Kaisha),<br>24 October, 1995 (24.10.95),<br>Full text; all drawings<br>(Family: none) | 4-14,16 |
| Y | JP 9-120464 A  (Matsushita Electric<br>Industrial Co., Ltd.),<br>06 May, 1997 (06.05.97),<br>Full text; all drawings<br>(Family: none) | 4-14,16 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>20 August, 2004 (20.08.04) | Date of mailing of the international search report<br>07 September, 2004 (07.09.04) |
|---|---|
| Name and mailing address of the ISA/<br>Japanese Patent Office | Authorized officer |
| Facsimile No. | Telephone No. |

Form PCT/ISA/210 (second sheet) (January 2004)

<div align="center">

**INTERNATIONAL SEARCH REPORT**

</div>

| | International application No. |
|---|---|
| | PCT/JP2004/006940 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 6-312007 A  (Shin'ichi BEPPU),<br>08 November, 1994 (08.11.94),<br>Full text; all drawings<br>(Family: none) | 7-16 |
| Y. | JP 7-328095 A  (Tomonori KUWABARA),<br>19 December, 1995 (19.12.95),<br>Full text; all drawings<br>(Family: none) | 7-16 |
| Y | Microfilm of the specification and drawings<br>annexed to the request of Japanese Utility<br>Model Application No. 123421/1989 (Laid-open<br>No. 64003/1991)<br>(Isao MITOMO),<br>21 June, 1991 (21.06.91),<br>Full text; all drawings<br>(Family: none) | 7-16 |
| Y | JP 2001-75705 A  (Toshiba Corp.),<br>23 March, 2001 (23.03.01),<br>Full text; all drawings<br>(Family: none) | 9-16 |
| Y | JP 2001-249753 A  (Sharp Corp.),<br>14 September, 2001 (14.09.01),<br>Full text; all drawings<br>(Family: none) | 9-16 |
| Y | JP 9-149915 A  (Aiphone Co., Ltd.),<br>10 June, 1997 (10.06.97),<br>Full text; all drawings<br>(Family: none) | 13,14,16 |

Form PCT/ISA/210 (continuation of second sheet) (January 2004)

## INTERNATIONAL SEARCH REPORT

International application No.

PCT/JP2004/006940

| Box No. II    Observations where certain claims were found unsearchable (Continuation of item 2 of first sheet) |
|---|

This international search report has not been established in respect of certain claims under Article 17(2)(a) for the following reasons:

1. ☒ Claims Nos.: 17 and 18
   because they relate to subject matter not required to be searched by this Authority, namely:

   A method of applying skin sensory stimulation described in claims 17 and 18 corresponds to a method of treating a human body by operative or medial treatment.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III    Observations where unity of invention is lacking (Continuation of item 3 of first sheet) |
|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐    The additional search fees were accompanied by the applicant's protest.

☐    No protest accompanied the payment of additional search fees.

Form PCT/ISA/210 (continuation of first sheet (2)) (January 2004)